# EUROPEAN PATENT SPECIFICATION

(11) **EP 2 921 193 B1**
(45) Date of publication and mention of the grant of the patent: **21.08.2019**
(21) Application number: 14001032.3
(22) Date of filing: 20.03.2014
(51) Int. Cl.: A61M 25/00

(54) **Multiflexible catheter tube**
Mehrfach flexibler Katheterschlauch
Tube de cathéter multiflexible

(43) Date of publication of application: 23.09.2015
(73) Proprietor: Willy Rüsch GmbH, 71394 Kernen-Rommelshausen (DE)
(72) Inventor: Morgan, Tierney, Ferbane, Co. Offaly (IE); Kelly, Ronald John, Oranmore, Go Galway (IE)
(74) Representative: Grünecker Patent- und Rechtsanwälte PartG mbB

(56) References cited:
- EP-A1- 2 364 746
- WO-A1-94/01160
- WO-A1-97/32623
- WO-A1-2010/080715
- WO-A2-2007/028058
- US-A- 5 573 520
- US-A1- 2005 049 545
- US-A1- 2006 084 939
- US-A1- 2007 066 900

## Description

The present invention refers to a urinary catheter for introduction in the human urethra with an inner catheter tube and an outer catheter tube which tightly surrounds the inner catheter tube.

Furthermore, the present invention also refers to a method for producing such a urinary catheter.

Catheters are long tubular devices used for insertion into body lumens, for example, the urethra. However, other catheter types, for example epidural catheters, may also be introduced through solid tissue. Catheters generally include at least one passageway over their length.

As catheters are guided through body lumens, it is generally desirable that the catheter is flexible to follow the tortuous body lumen and to reduce trauma. Furthermore, the catheter must also possess sufficient strength or rigidity so that it can be pushed through the body lumen. Catheters will typically be advanced by pushing them manually forward so that the advancement force is transmitted axially along the length of the catheter. In order to transmit this force without kinking or buckling, the catheter must possess sufficient column strength to resist the compressive forces of advancement.

Such a catheter is for example known from document US 5,947,940. This document discloses a strong, thin walled, highly flexible catheter comprising an outer tubular cover and at least one helical reinforcing member which is inserted in the outer cover. The helical reinforcing member consists of a stiff band which is helically wound to form the helical reinforcing member. Therefore, between the single turns of the helical reinforcing member, gaps exist. For producing the catheter, the helical reinforcing band is wound upon a core which is subsequently removed. After that, the outer tubular layer is applied by extrusion or die-coating.

EP 2 364 746 A1 shows a catheter, preferably an ultra-sound catheter for observing the inside of a body cavity or lumen. The catheter comprises an outer tube which is closely attached to an inner (intermediate) tube. The outer tube and the inner tube are non-metallic; the inner tube is made of a harder material than the outer tube. The inner tube comprises a spiral cut which is a through cut and passes radially completely through the inner tube.

WO 2007/028058 A2 discloses an intravascular catheter with an adjustable stiffness. The catheter includes a hypotube which is disposed within a polymeric layer and which has spirally aligned cuts or curves separating adjacent bridge portions.

US 2006/084939 A1 refers to an articulation segment for a cryo-catheter. The articulation segment includes a tube-shaped member which is formed with a helically-shaped gap and which is covered by a flexible coating.

US 5,573,520 A shows an apparatus for use as a catheter, a guidewire, a catheter sheath or a drug infusion catheter/guidewire. The apparatus comprises a tube encased in a low friction material and having a plurality of slots which can have a helical shape.

US 2007/066900 A1 discloses an intravascular ultra-sound catheter having an elongated shaft with an outer polymer layer, an inner polymer layer and an metallic intermediate layer with spiral cuts.

WO 97/32623 A1 shows an elongated member for an arterial or venous cardiopulmonary bypass cannula. The member can consist of two layers and is produced by inflating an outer tube and then positioning an inner tube inside the outer tube.

WO 2010/080715 A1 discloses an infusion device comprising a catheter body with a torsion spring which can be coated over by over moldinglt is the object of the present invention to provide a reinforced urinary catheter and a method for producing this urinary catheter which overcome at least partially the disadvantages of the devices and methods known so far and which can be easily introduced in a body lumen by a patient or user themselves and has nevertheless a good kinking resistance and pushability and can be easily manufactured.

For this purpose, the outer catheter tube comprises a hydrophilic coating at least along its insertable length, the inner catheter tube and the outer catheter tube are non-metallic, the inner catheter tube is made of a stiffer material than the outer catheter tube, the inner catheter tube is provided with a helical cut which completely penetrates the wall of the inner catheter tube and adjacent parts of the inner catheter tube which are separated by the helical cut abut each other so that there is no space between them.

Due to the helical cut, the inner catheter tube is easily bendable and can therefore be easily introduced in a tortuous body lumen, for example the urethra, but still has sufficient stiffness in its longitudinal direction to insert and push the catheter forward. When pushing the catheter forward, the parts of the inner catheter tube separated by the helical cut abut each other and the force in the axial or forward direction is transmitted and transformed in a forward movement of the catheter. The depth of the helical cut corresponds to at least the wall thickness of the inner catheter tube. When the cut completely separates the wall, good bending properties and good pushability is achieved. The outer catheter tube which surrounds the inner catheter tube ensures the impermeability of the catheter and provides a continuous outer surface of the catheter. This urinary catheter can be used for intermittent self-catheterisation by handicapped users and is very easy in handling and can be easily inserted without causing harm to the urethra. The hydrophilic coating further reduces friction when introducing the catheter into a body lumen. Furthermore, the urinary catheter is MRI compatible. The inner catheter tube provides the stiffness in radial and axial direction which is necessary for pushing the catheter forward and for avoiding kinking of the catheter but still has a high bendability due to the helical cut whereas the outer catheter tube ensures the impermeability and low friction of the catheter.

In a further embodiment it can be provided that the pitch of the helical cut varies along the length of the inner catheter tube. Therefore, the stiffness and the bending radius of the catheter vary along the length of the catheter. In the tip region of the catheter, that is the region of the catheter which is first inserted in the body lumen, the helical cut has a small pitch so that the single turns of the helical cut lay close together. This leads to a small bending radius and a relatively low stiffness in this region. In the other regions of the catheter the pitch - and therewith the bending radius - can be larger and the catheter is stiffer.

It can further be provided that only the distal end of the inner catheter tube is provided with the helical cut. The distal end of the catheter then has the desired bendability whereas the proximal part of the catheter tube can be used as a handle and improves the pushability and handling of the catheter. In this context the term "distal" means the part of the catheter which is directed towards a user's body when introducing the catheter, the term "proximal" refers to the other end of the catheter.

In another embodiment the inner catheter tube and the outer catheter tube may be transparent. The catheter tubes then provide a visual indication, of fluid presence and movement within the catheter, for example.

In yet another embodiment the inner and outer catheter tubes may contain holes for fluid introduction or removal through the catheter.

In another embodiment the tensile performance of the catheter may be improved with the addition of a longitudinal reinforcement such as fibres provided between the inner catheter tube and the outer catheter tube or within the outer catheter tube.

Furthermore, the above mentioned object is also solved by a method for producing a urinary catheter as described above and comprising the following steps:
- Applying a pressure differential between the inside and the outside of the outer catheter tube so that its internal diameter is enlarged,
- Inserting the inner catheter tube with the helical cut in the enlarged outer catheter tube,
- Releasing the pressure differential so that the outer catheter tube contracts and tightly surrounds the inner catheter tube.

Commonly known methods for producing reinforced catheters such as over extrusion and dip coating can be difficult to control and often require long periods of time for the soft outer catheter tube to stabilize. The present method offers a safe and easy manufacturing method and leads to a product that can be immediately packed and shipped.

In a variant of the method, it can be provided that the pressure differential on the outer catheter tube is applied via the injection of a pressurized fluid, for example air or water. Therefore, only harmless ingredients are used for the manufacturing so that special safety precautions, which are necessary when handling for example solvents, can be avoided.

Furthermore, it can be provided that the pressure applied on the outer catheter tube lies in a range of 5 to 30 bar. This leads to a sufficient increase in the inner diameter of the outer catheter tube. However, the pressure force is not strong enough to damage the outer catheter tube.

In still another variant of the method, the outer catheter tube is inserted in a cylindrical housing before the internal pressure is applied. This leads to a uniform inflation of the outer catheter tube so that any "ballooning effect", wherein some regions of the catheter tube are larger than others, is avoided.

In addition to the use of a pressure differential, the outer tube length may be managed throughout the assembly process therefore providing an additional compression or tension between the inner and outer tube in the formed catheter. It is for example possible to apply tension to the outer catheter tube during inflation so the tube is stretched. Due to the stretching in the longitudinal direction, the outer catheter tube compresses the inner catheter tube with the helical cut and therefore influences the behaviour of the assembled, finished catheter.

In another very easy method for producing a urinary catheter as described above, the outer catheter tube is over-moulded on the inner catheter tube and encapsulates at least the region of the inner catheter tube which is provided with the helical cut. The over-moulding can be profiled to create a smooth crossing-profile of the device and can comprise surface apertures to facilitate fluid transfer to and from the inner catheter tube. Pending on the over-moulding material and the material of the inner catheter tube, the inner catheter tube with the helical cut can be adhered to the over-moulded outer catheter tube or not.

In the following, the invention is described in more detail with the aid of drawings:
Figure 1 shows a urinary catheter according to the present invention,
Figure 2 shows a cross section of the catheter tube of the catheter shown in Figure 1,
Figure 3 shows the inner catheter tube of the catheter in Figures 1 and 2,
Figure 4 shows the stiffness of a catheter along its longitudinal direction,
Figure 5 shows another embodiment of the urinary catheter and
Figure 6 shows a device for manufacturing the catheter of Figures 1 to 3.

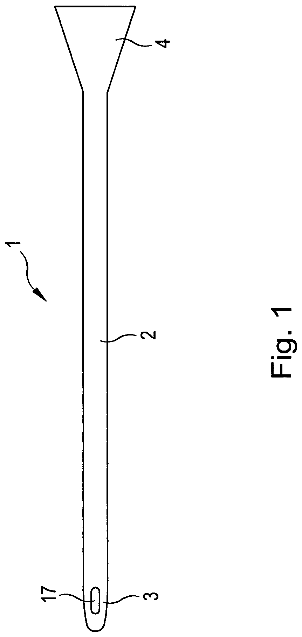

Figure 1 shows a urinary catheter 1 according to the present invention. The urinary catheter 1 comprises a catheter tube 2 which is provided at one end with a catheter tip 3 with at least one eyelet or opening 17 and the other end with a funnel 4. The catheter 1 defines an internal passageway which extends from the catheter tip 3 to the other end of the catheter 1 with the funnel 4. Through this passageway, liquid can be inserted through the funnel 4 and the tip 3 into a body lumen or can be drained from a body lumen through the catheter tip 3, the internal passageway and the other end with the funnel 4 out of the body.

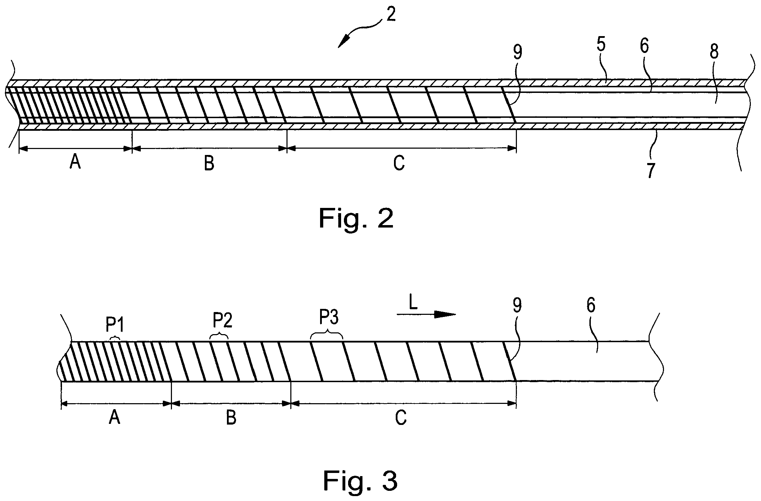

Figure 2 shows a cross section through the catheter tube 2 as shown in Figure 1. The catheter tube 2 comprises an outer catheter tube 5 and an inner catheter tube 6. The outer catheter tube 5 forms the outer surface 7 of the catheter tube 2. The inner catheter tube 6 defines the internal passageway 8 of the catheter. The inner catheter tube 6 is provided with a helical cut 9. In Figure 2, the helical cut 9 does not extend over the complete length of the catheter tube 2 but is only provided along the insertable length of the catheter tube 2. The term "insertable length" defines the length of the catheter tube which is intended and adapted for the insertion into a body lumen.

In Figure 2, the helical cut 9 has different pitches. In the front region A, the pitch of the helical cut 9 is small. In the following region B, the pitch of the helical cut 9 is increased and is even larger in the following region C. Therefore, the inner catheter tube 6 has a different bendability and a different bending radius along its length. However, the pitch of the helical cut may also be kept constant along a part or the entire length of the inner catheter tube.

The helical cut 9 as shown in Figure 2 extends completely through the wall of the inner catheter tube 6. This means that the depth of the helical cut is at least as large as the thickness of the wall of the inner catheter tube 6.

The inner catheter tube 6 and the outer catheter tube 5 are made of different materials. The material of the inner catheter tube 6 is stiffer than the material of the outer catheter tube 5. The inner catheter tube 6 can, for example, be made of polyethylene or nylon, the outer catheter tube 5 can be made of polyurethane. The inner catheter tube 6 therefore has a higher stiffness than the outer catheter tube and transmits the axial forces acting along the length of the catheter 1 when introducing the catheter in a body lumen. The catheter 1 can thus be easily inserted in the body lumen. As the inner catheter tube 6 is provided with a helical cut 9 and not with a helical groove, adjacent parts of the inner catheter tube 6 which are separated by the helical cut 9 still abut at each other and there is no space between them. Therefore, the axial forces can be transmitted without compressing the inner catheter tube 6. As both catheter tubes, the inner catheter tube and the outer catheter tube, are preferably made of non-metallic material, preferably plastics, the catheter is MRI-compatible.

The material of the outer catheter tube 5 is softer than the material of the inner catheter tube 6. The outer catheter tube 5 tightly surrounds the inner catheter tube 6 and therefore leads to a liquid impermeable catheter tube. Due to the soft material used for the outer catheter tube 5, the bending properties of the catheter 1 and the catheter tube 2 are not affected and the whole catheter 1 can easily be inserted along the tortuous body lumen. Furthermore, the coefficient of friction of the outer catheter tube 5 is preferably very small. For example, the outer catheter tube can be coated with a hydrophilic coating. This helps to easily introduce the 1 catheter into a body lumen. No harm or trauma to the body lumen is caused.

Figure 3 shows a part of the inner catheter tube 6. The helical cut 9 which extends along the length L of the catheter tube can be clearly seen. In the front region A of the inner catheter tube 6, which is the region which is at first introduced into the body lumen, the helical cut 9 has a first pitch P1. In the following region B of the inner catheter tube, the helical cut 9 has a second pitch P2. In the example shown, pitch P2 is twice as large as pitch P1. In the region C which follows region B, the helical cut 9 has a third pitch P3. Pitch P3 is approximately twice as large as pitch P2. Therefore, the tip region of the catheter has a very small bending radius which allows insertion of the tip for example in the bladder of a user. In the following regions, the bending radius of the catheter 1 and the stiffness of the catheter 1 increase. Furthermore, the stiffness of the catheter 1 can be fine-tuned at the interface of the regions A and B and at the interface of the regions B and C by the introduction of a transient pitch region creating a gradual increase in pitch, i.e. from pitch P2 to pitch P3.

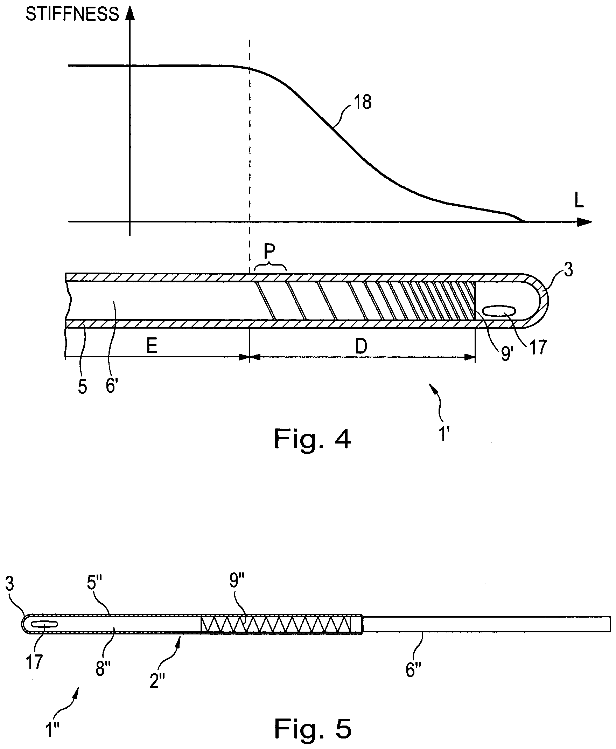

Figure 4 shows another embodiment of a urinary catheter 1' according to the present invention and a diagram showing the change of the flexural stiffness of the catheter 1' along the catheter length L. The urinary catheter 1' basically corresponds to the urinary catheter as described above. In the following, only the differences are disclosed. In Figure 4, only the front part of the urinary catheter 1' is shown, the outer catheter tube 5 is shown in cross section; the inner catheter tube 6' is not cut. The urinary catheter 1' comprises an outer catheter tube 5 and an inner catheter tube 6'. The inner catheter tube 6' is provided with a helical cut 9'. The helical cut 9' extends only along the distal section D of the catheter 1' which is arranged near the catheter tip 3. In the remaining or proximal part E of the urinary catheter 1' the inner catheter tube 6' is not slotted or cut. The inner catheter tube 6' ends shortly before the catheter tip 3. The catheter tip 3 is provided with an eyelet 17. The pitch P of the helical cut 9' varies along the length L of the urinary catheter 1'. Near the tip 3 of the urinary catheter 1', the pitch P of the helical cut 9' is very small and increases in the direction towards the proximal end of the urinary catheter 1'. Preferably, the pitch P increases continuously. As shown in the diagram above the urinary catheter 1' with the graph 18, the stiffness of the urinary catheter 1' varies along the catheter length L. In the proximal region E, where the inner catheter tube 6' is not provided with a helical cut, the stiffness is on a constant high level. In the region D of the inner catheter tube 6' with the helical cut 9' the stiffness of the urinary catheter 1' decreases until it reaches a minimum value at the catheter tip 3.

Figure 5 shows a further embodiment of a urinary catheter 1". The urinary catheter 1" as shown in Figure 5 basically corresponds to the other embodiments as described above. In the following, only the differences are described. The urinary catheter 1" comprises an outer catheter tube 5" and an inner catheter tube 6". The inner catheter tube 6" comprises a helical cut 9" at its front end or distal end which is closest to the catheter tip 3. The helical cut 9" does not extend over the complete length of the urinary catheter 1". Furthermore, the pitch of the helical cut 9" is constant. The outer catheter tube 5" is over-moulded on the outer catheter tube 6". The outer catheter tube 5" does not extend along the complete length of the catheter 1" but only encapsulates the part of the inner catheter tube 6" which is provided with the helical cut 9". Furthermore, the outer catheter tube 5" extends to the distal end of the urinary catheter 1" and forms the catheter tip 3 as well as the eyelet 17. In this tip region, the urinary catheter 1" is not provided with an inner catheter tube. It is also possible that the outer catheter tube is a component that is positioned and attached to the inner catheter tube so that it at least encapsulates the helical cut section of the inner catheter tube. The outer catheter tube, which can be over-moulded or an attached component, is preferably profiled to create a smooth outer surface on the urinary catheter. Depending on the material of the attached component or on the over-moulding material and the material of the inner catheter tube, the inner catheter tube with the helical cut may be adhered to the outer catheter tube or not.

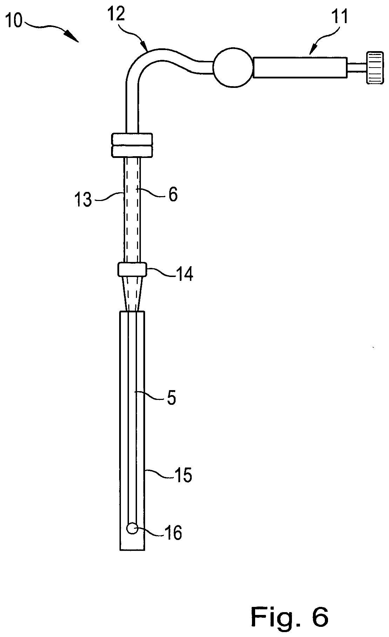

Figure 6 shows a device 10 for producing the catheter 1 as described above. The device 10 comprises an apparatus 11 for pressurizing a fluid, a tube 12 which connects the apparatus 11 to a holding mechanism for holding the outer catheter tube 5, an insertion mechanism 13 for holding and inserting the inner catheter tube 6, a cylindrical housing 15 and an end cap 16 for tightly closing the outer end of the outer catheter tube 5.

In the following, the method for producing the catheter is described with the aid of Figure 6. The outer catheter tube 5 made of a soft material is placed on the holder 14. The free end of the outer catheter tube 5 is closed with the end cap 16 so that the outer catheter tube 5 forms a closed system together with the holder 14 and the end cap 16. Then, the cylindrical housing 15 is placed around the outer catheter tube 5. The inner catheter tube 6 with the helical cut 9 is inserted in the insertion mechanism 13. The outer catheter tube 5 is then connected via the holder 14, the insertion mechanism 13, and the tube 12 with the pressure apparatus 11 in a fluid tight manner. In the next step, a pressurized fluid, for example air or water, which is pressurized via the pressure apparatus 11, is injected in the outer catheter tube 5 so that an internal pressure or a pressure differential is applied on the outer catheter tube 5. This leads to an increase of the inner diameter of the outer catheter tube 5. Then, the inner catheter tube 6 with the helical cut 9 is inserted in the expanded outer catheter tube 5 via the insertion mechanism 13. When the inner catheter tube 6 is completely inserted in the outer catheter tube 5, the pressure applied on the outer catheter tube 5 is released and the outer catheter tube 5 contracts and tightly surrounds the inner catheter tube 6.

The pressure applied on the outer catheter tube 5 preferably lies in a range from 5 to 30 bar. In this range, the increase in inner diameter of the outer catheter tube 5 is sufficient to insert the inner catheter tube 6, however, the outer catheter tube 5 is not harmed and still contracts after the release of the pressure. This range of pressure is the optimal range for a urinary catheter. However, for other catheter designs or other applications, for example micro-catheter assemblies or larger diameter devices, other ranges of pressure are desirable. The reinforced catheter tube 2 with the inner catheter tube 6 and the outer catheter tube 5 produced as described above can then be used for manufacturing catheters.

In another simple embodiment for the manufacturing method, the catheter 1" as described above can be produced by over-moulding the inner catheter tube 6" at least in the region where it is provided with the helical cut 9" with a second, softer material to produce the outer catheter tube 5".

The catheters as described above comprising an inner catheter tube with a helical cut and an outer catheter tube tightly surrounding the inner catheter tube can be used in a variety of catheter types. Apart from urinary catheters, epidural catheters, introducers and introducer guide catheters are often reinforced.

All catheters as described above may comprise transparent inner and outer catheter tubes so that a visual indication for example for fluid presence or movement within the catheter is provided. Furthermore, the inner catheter tube and the outer catheter tube may contain holes for fluid introduction or removal through the catheter. The catheters can be provided with a longitudinal reinforcement such as fibres provided between the inner catheter tube and the outer catheter tube or within the outer catheter tube. In this case, the tensile performance of the catheter is improved.

### Reference Numbers

- 1; 1'; 1": Urinary catheter
- 2; 2": Catheter tube
- 3: Catheter tip
- 4: Funnel
- 5; 5": Outer catheter tube
- 6; 6'; 6": Inner catheter tube
- 7: Outer surface catheter tube
- 8: Internal passageway
- 9; 9'; 9": Helical cut
- 10: Manufacturing device
- 11: Pressure apparatus
- 12: Tube
- 13: Insertion mechanism
- 14: Holding mechanism
- 15: Cylindrical housing
- 16: End cap
- 17: Eyelet
- 18: Graph
- L: Longitudinal direction of the catheter
- P: Pitch of helical cut
- P1: First pitch
- P2: Second pitch
- P3: Third pitch
- A; B; C; D; E: Regions of the catheter

## Claims

1. Urinary catheter (1; 1'; 1") for the introduction in the human urethra with an inner catheter tube (6; 6'; 6") and an outer catheter tube (5; 5") which tightly surrounds the inner catheter tube (6; 6'; 6"), wherein the outer catheter tube (5; 5") comprises a hydrophilic coating at least along its insertable length, the inner catheter tube (5; 5") and the outer catheter tube (6; 6'; 6") are non-metallic, the inner catheter tube (6; 6'; 6") is made of a stiffer material than the outer catheter tube (5; 5"), the inner catheter tube (6; 6'; 6") is provided with a helical cut (9; 9'; 9"), which completely penetrates the wall of the inner catheter tube (6; 6'; 6") and adjacent parts of the inner catheter tube which are separated by the helical cut abut each other so that there is no space between them.

2. Urinary catheter according to claim 1, **characterized in that** the pitch of the helical cut (9; 9') varies along the length of the inner catheter tube (6; 6').

3. Urinary catheter according to at least one of claims 1 or 2, **characterized in that** only the distal end of the inner catheter tube (6; 6'; 6") is provided with the helical cut (9; 9'; 9").

4. Urinary catheter according to any of the preceding claims, **characterized in that** the inner catheter tube (5; 5") and the outer catheter tube (6; 6'; 6") are transparent.

5. Urinary catheter according to any of the preceding claims, **characterized in that** the catheter (1; 1'; 1") comprises a longitudinal reinforcement.

6. Method for producing a urinary catheter (1) according to any of the claims 1 to 5, **characterized by** the following steps:
• Applying a pressure differential between the inside and the outside of the outer catheter tube (5) so that its internal diameter is enlarged,
• Inserting the inner catheter tube (6) with the helical cut (9) in the enlarged outer catheter tube (5),
• Releasing the pressure differential so that the outer catheter tube (5) contracts and tightly surrounds the inner catheter tube (6).

7. Method according to claim 6, **characterized in that** the pressure differential on the outer catheter tube (5; 5") is applied via the injection of a pressurized fluid, for example air or water.

8. Method according to claim 6 or 7, **characterized in that** the pressure differential applied on the outer catheter tube (5) lies in a range of 5 to 30 bar.

9. Method according to at least one of claims 6 to 8, **characterized in that** the outer catheter tube (5) is inserted in a cylindrical housing (15) before the pressure differential is applied.

10. Method for producing a urinary catheter (1") according to any of the claims 1 to 5 **characterized in that** the outer catheter tube (5") is over-moulded on the inner catheter tube (6") and encapsulates at least the region of the inner catheter tube (6") which is provided with the helical cut (9").

## Patentansprüche

1. Blasenkatheter (1; 1'; 1") zum Einführen in die menschliche Harnröhre mit einem inneren Katheterschlauch (6; 6'; 6") und einem äußeren Katheterschlauch (5; 5"), der den inneren Katheterschlauch (6; 6'; 6") eng umgibt, wobei der äußere Katheterschlauch (5; 5") zumindest entlang seiner einführbar Länge eine hydrophile Beschichtung aufweist, der innere Katheterschlauch (5; 5") und der äußere Katheterschlauch (6; 6'; 6") nichtmetallisch sind, der innere Katheterschlauch (6; 6'; 6") aus einem steiferen Material als der äußere Katheterschlauch (5; 5") ist, der innere Katheterschlauch (6; 6'; 6") mit einem schraubenförmigen Schnitt (9; 9'; 9") versehen ist, der die Wandung des inneren Katheterschlauchs (6; 6'; 6") vollständig durchdringt und benachbarte Teile des inneren Katheterschlauch, die durch den schraubenförmigen Schnitt getrennt sind, aneinander anliegen, so dass kein Zwischenraum zwischen ihnen ist.

2. Blasenkatheter nach Anspruch 1, **dadurch gekennzeichnet, dass** sich die Steigung des schraubenförmigen Schnitts (9; 9') entlang der Länge des inneren Katheterschlauch (6; 6') ändert.

3. Blasenkatheter nach mindestens einem der Ansprüche 1 oder 2, **dadurch gekennzeichnet, dass** nur das distale Ende des inneren Katheterschlauch (6; 6'; 6") mit dem schraubenförmigen Schnitt (9; 9'; 9") versehen ist.

4. Blasenkatheter nach irgendeinem der vorhergehenden Ansprüche, **dadurch gekennzeichnet, dass** der innere Katheterschlauch (5; 5") und der äußere Katheterschlauch (6; 6'; 6") transparent sind.

5. Blasenkatheter nach irgendeinem der vorhergehenden Ansprüche, **dadurch gekennzeichnet, dass** der Katheter (1; 1'; 1") eine Verstärkung in Längsrichtung umfasst.

6. Verfahren zum Herstellen eines Blasenkatheters (1) nach einem der Ansprüche 1 bis 5, **gekennzeichnet durch** die folgenden Schritte:
- Aufbringen eines Druckunterschieds zwischen der Innenseite und der Außenseite des äußeren Katheterschlauch (5), so dass sein Innendurchmesser vergrößert ist,
- Einführen des inneren Katheterschlauch (6) mit dem schraubenförmigen Schnitt (9) in den vergrößerten äußeren Katheterschlauch (5),
- Aufheben des Druckunterschieds, so dass sich der äußere Katheterschlauch (5) zusammenzieht und den inneren Katheterschlauch (6) eng umgibt.

7. Verfahren nach Anspruch 6, **dadurch gekennzeichnet, dass** der Druckunterschied auf den äußeren Katheterschlauch (5; 5") durch Einspritzen eines unter Druck stehenden Fluids, beispielsweise Luft oder Wasser, aufgebracht wird.

8. Verfahren nach Anspruch 6 oder 7, **dadurch gekennzeichnet, dass** der auf den äußeren Katheterschlauch (5) aufgebrachte Druckunterschied in einem Bereich von 5 bis 30 bar liegt.

9. Verfahren nach mindestens einem der Ansprüche 6 bis 8, **dadurch gekennzeichnet, dass** der äußere Katheterschlauch (5) vor dem Aufbringen des Druckunterschieds in ein zylindrisches Gehäuse (15) eingeführt wird.

10. Verfahren zur Herstellung eines Blasenkatheters (1") nach einem der Ansprüche 1 bis 5, **dadurch gekennzeichnet, dass** der äußere Katheterschlauch (5") über den inneren Katheterschlauch (6") gespritzt ist und zumindest den Bereich des inneren Katheterschlauch (6"), der mit dem schraubenförmigen Schnitt (9") versehen ist, einkapselt.

## Revendications

1. Cathéter urinaire (1 ; 1' ; 1") destiné à l'introduction dans l'urètre humain avec un tube de cathéter interne (6 ; 6' ; 6") et un tube de cathéter externe (5 ; 5") qui entoure fermement le tube de cathéter interne (6 ; 6' ; 6"), le tube de cathéter externe (5 ; 5") comprenant un revêtement hydrophile au moins le long de sa longueur insérable, le tube de cathéter interne (5 ; 5") et le tube de cathéter externe (6 ; 6' ; 6") étant non métalliques, le tube de cathéter interne (6 ; 6' ; 6") étant constitué d'un matériau plus rigide que le tube de cathéter externe (5 ; 5"), le tube de cathéter interne (6 ; 6' ; 6") étant muni d'une encoche hélicoïdale (9 ; 9' ; 9"), qui pénètre entièrement la paroi du tube de cathéter interne (6 ; 6' ; 6") et des parties adjacentes du tube de cathéter interne qui sont séparées par l'encoche hélicoïdale viennent en butée l'une contre l'autre de sorte qu'il n'existe pas d'espace entre elles.

2. Cathéter urinaire selon la revendication 1, **caractérisé en ce que** le pas de l'encoche hélicoïdale (9 ; 9') varie le long de la longueur du tube de cathéter interne (6 ; 6').

3. Cathéter urinaire selon au moins l'une des revendications 1 ou 2, **caractérisé en ce que** uniquement l'extrémité distale du tube de cathéter interne (6 ; 6' ; 6") est munie de l'encoche hélicoïdale (9 ; 9' ; 9").

4. Cathéter urinaire selon l'une quelconque des revendications précédentes, **caractérisé en ce que** le tube de cathéter interne (5 ; 5") et le tube de cathéter externe (6 ; 6' ; 6") sont transparents.

5. Cathéter urinaire selon l'une quelconque des revendications précédentes, **caractérisé en ce que** le cathéter (1 ; 1' ; 1") comprend un renfort longitudinal.

6. Procédé de production d'un cathéter urinaire (1) selon l'une quelconque des revendications 1 à 5,
**caractérisé par** les étapes suivantes :
• application d'un différentiel de pression entre l'intérieur et l'extérieur du tube de cathéter externe (5) de sorte que son diamètre interne est élargi,
• insertion du tube de cathéter interne (6) avec l'encoche hélicoïdale (9) dans le tube de cathéter externe élargi (5),
• relâchement du différentiel de pression de sorte que le tube de cathéter externe (5) se contracte et entoure fermement le tube de cathéter interne (6).

7. Procédé selon la revendication 6, **caractérisé en ce que** le différentiel de pression sur le tube de cathéter externe (5 ; 5") est appliqué par l'intermédiaire de l'injection d'un liquide sous pression, par exemple d'air ou d'eau.

8. Procédé selon la revendication 6 ou 7, **caractérisé en ce que** le différentiel de pression appliqué sur le tube de cathéter externe (5) s'étend dans une plage de 5 à 30 bars.

9. Procédé selon au moins l'une des revendications 6 à 8, **caractérisé en ce que** le tube de cathéter externe (5) est inséré dans un logement cylindrique (15) avant que le différentiel de pression soit appliqué.

10. Procédé de production d'un cathéter urinaire (1") selon l'une quelconque des revendications 1 à 5 **caractérisé en ce que** le tube de cathéter externe (5") est surmoulé sur le tube de cathéter interne (6") et encapsule au moins la région du tube de cathéter interne (6") qui est prévue avec l'encoche hélicoïdale (9").
